# EUROPEAN PATENT SPECIFICATION

(11) **EP 1 911 411 B1**
(45) Date of publication and mention of the grant of the patent: **21.07.2010**
(21) Application number: 07118314.9
(22) Date of filing: 11.10.2007
(51) Int. Cl.: A61B 19/02, A61M 25/06, A61M 27/00

(54) **Medical needle puller**
Medizinischer Nadelpuller
Crochet d'aiguille médicale

(30) Priority: 11.10.2006 JP 2006277123
(43) Date of publication of application: 16.04.2008
(73) Proprietor: Covidien AG, 8212 Neuhausen am Rheinfall (CH)
(72) Inventor: Itoh, Akira, Shizuoka (JP); Baba, Yasuyuki, Shizuoka (JP); Makino, Masanori, Shizuoka (JP); Koike, Kazuhiro, Shizuoka (JP)
(74) Representative: Körber, Martin Hans

## Description

### [Technical Field]

The present invention relates to a medical needle puller used when pulling out a medical needle used to insert a drainage tube to a postsurgical wound site from the wound site.

### [Background Art]

After the surgery, a procedure in which a drainage tube is inserted into a wound site to drain a wound fluid secreted from the wound site has been traditionally practiced. In the foregoing case, the medical needle is attached to the tip part of the drainage tube, and the medical needle punctures through a patient body to place the rear end side portion of the drainage tube at the wound site. And, after the medical needle is removed from the tip part of the drainage tube, a hose end extending from a trapping container through a pump is connected to the tip part and then the pump is actuated to aspirate the wound fluid to the inside of the trapping container (See for example, Patent Literature 1).
[Patent Literature 1] Unexamined Patent Publication Number H04-261670

Prior art literature presents a series of medical needle pullers and protectors respectively which attempt to cover a needle properly before being pulled out of a wound site.

Document WO 95/07663 discloses a method of removing a distal end portion (medical needle) from a proximal portion of a trocar assembly which includes a means for releasing the distal end portion without requiring the user to grasp the distal end portion. Said means comprises a pair of needle coating members hinge-connected at one edge and folded together, between which a needlepoint accommodation part for accommodating the aforementioned puncture part is formed.

Document [US-5-090-564] discloses a disposable hypodermic needle sheath and remover which is designed as a disposable two-part container, having two halves. The container can be placed in an open position and a used needle placed into its ribs. The ribs have needle receiving grooves or channels which are disposed within the container and will grasp the needle when the container is closed.

Document [US-2006/0138004] relates to a multipurpose device for the safe handling of sharp surgical implements and comprises a molded thermoplastic body that includes a container portion and a scalpel holder section. The scalpel holder section supports a scalpel or scalpels with upstanding rips having a lengthwise deep groove to be cut by a scalpel blade and thereby accommodating the scalpel.

Document [US- 5-188-612] discloses a disposable phlebotomist protector device for enclosing a used needle assembly. The device includes a pair of housing elements each having an end portion and an integral elongate portion. At least one end portion has cannula inset elements for retaining the cannula of a needle assembly when the two housing elements are in an open position relative to each other. The housing elements include locking elements designed to engage the two housing elements to form a closed housing that encloses the needle assembly.

Document [EP-1-323-442] presents a needle cover capable of storing an entire needle comprising a top side opening part for taking therein an entire winged needle having a flexible tube connected thereto and a base side opening part allowing the flexible tube to pass therethrough. The cover consists of a pair of hinged coating means that engage with each other to hold the needle assembly.

### [Disclosure of the Invention]

When the foregoing conventional medical needle is pulled out from a patient body, methods such as a method in which the medical needle is grasped directly by hand and a method in which the medical needle is clamped by a pair of forceps to pull out the needle have been practiced. However, there are drawbacks when pulling out the medical needle from a patient body by hand or the pair of forceps, for example a problem that the medical needle tends to be slippery and cannot easily be pulled out, or a problem that user's hand may accidentally touch the medical needle and may cause injuries.

Moreover, when discarding used medical needles, the needlepoint portion must be covered with a cap or the like, otherwise safety problems may result.

The present invention is made in consideration of the aforementioned foregoing situations and the purpose is to provide a medical needle puller that can be easily pulled out from a patient body while improving safety.

To achieve the aforementioned purpose, the medical needle puller according to the present invention is structurally characterised in that: a connection part for connecting a drainage tube placed at a postsurgical wound site is formed at the rear end part; a puncture part is formed at the tip part, and at the same time, wherein the medical needle puller is attached to the medical needle in which an engaging stepped part is formed at the back side of the puncture part, comprising an engaging part engaging to the aforementioned engaging stepped part and a needlepoint coating part for coating the aforementioned puncture part comprising a pair of coating members hinge-connected at one edge and folded together, between which a needlepoint accommodation part for accommodating the aforementioned puncture part is formed, whereby said needlepoint accommodation part comprises a needlepoint accommodating concave part corresponding to the shape of the puncture part and a plane adapted to close said needlepoint accommodating concave part and bring said medical needle into contact with a wall surface of said needlepoint accommodating concave part.

The medical needle puller according to the present invention comprises the needlepoint coating part for coating the medical needle and the engaging part for engaging to the engaging stepped part formed at the back side of the puncture part in the medical needle. Consequently, when pulling out the medical needle from a body, the puller is attached to the puncture part of medical needle, the puller can be grasped and manipulated, thereby the medical needle can be easily pulled out from a patient body, preventing a risk of user's hand from slipping. At that time, since the puncture part of the medical needle is covered by the needlepoint coating part, a risk of user's hand accidentally touching the puncture part and causing injury is eliminated.

Moreover, the engaging part of the puller engages to the engaging stepped part of the medical needle, thereby the medical needle can be securely pulled out from a patient body by pulling the puller. In this case, the engaging stepped part may be structured by the concave part formed as depression in the outer peripheral surface of the medical needle, or may be structured by a protrusion part protruding from the outer peripheral surface of the medical needle, however, the engaging stepped part is preferably structured by the concave part in order to minimise the resistance when making a puncture with the medical needle. In addition, used medical needles can be discarded with the puller still attached to the puncture part. Consequently, needs for the removal of the puller from the medical needle and for the attachment of a special cap for disposal and the like to the puncture part are eliminated.

Moreover, the medical needle puller according to the present invention is characterised in that a gripper for pulling a puller by hand is provided when the medical needle is pulled out from a wound site together with the puller, while attaching the puller to the puncture part of the medical needle. Whereby, the medical needle can be pulled out from a body by grasping the gripper, and the medical needle can be easily as well as securely pulled out from a patient body.

Moreover, the medical needle puller according to the present invention is further structurally characterised in that the needlepoint coating part is formed so as to be extending along the extending direction of the puncture part, and that the gripper is comprised of the pair of protrusions extending to different directions respectively so as to be perpendicularly intersecting with the needlepoint coating part from both sides of the width direction of the needlepoint coating part. Whereby, the puller is T-shaped or cross-shaped, and the gripper consisting of the pair of protrusions can be grasped between fingers with the needlepoint coating part positioned, thereby a tighter hand grip force can be applied and the medical needle can be pulled out from a patient body more easily.

Moreover, the medical needle puller according to the present invention is further structurally characterised in that the engaging part is comprised of the concave part formed at a predetermined part of the jointed surface joined when the pair of coating members are folded together, passing through the needlepoint coating part from inside to outside.

Whereby, the pair of the coating members may be folded together so as to interleave the puncture part to attach the puller to the medical needle, thereby the puller can be easily attached to the medical needle. Moreover, the puller may now be easily engaged to the medical needle by simply positioning the concave part formed at the jointed surface of the pair of the coating members, allowing easier manipulation. Meanwhile, the needlepoint accommodation part may be structured in any size as long as is capable of accommodating the puncture part, however is preferably comprised of the concave part corresponding to the shape of the puncture part. Whereby, the puncture part stops jolting in the needlepoint accommodation part, and the puller can now be more solidly attached to the medical needle.

Moreover, the medical needle puller according to the present invention is further structurally characterised in that the pair of the coating members structuring the needlepoint coating part are provided with a chuck part for maintaining the pair of coating members in the folded condition. In this case, the chuck part may be a part capable of maintaining the pair of the coating members in the folded condition by engaging to the mutually engageable concave part, protrusions and the like. Whereby, the puller attached to the puncture part of the medical needle can no longer disengage from the puncture part and the medical needle can now be easily pulled out from a patient body, while improving safety as well. Moreover, the chuck part is preferably provided in the vicinity of the engaging part, hereby the effect can be further enhanced.

### [Best Mode for Carrying Out the Invention.]

### (The First Embodiment)

Hereinafter, the first embodiment of this invention will be descried in detail with reference to drawings. In Fig. 1 and Fig. 2, a metal medical needle 10 to which a puller 20 (see Fig. 4 and Fig. 5) according to the same embodiment is attached is shown. This medical needle 10 is comprised of a small diameter cylindrical needle's main body 11, a puncture part 12 formed at the tip side of the needle's main body 11, a connection part 13 formed at the rear end of the needle's main body 11, wherein an engaging stepped part 14 is formed between the needle's main body 11 and the puncture part 12, and a thin part 15 is formed at the tip side portion along the longitudinal direction of the needle's main body 11.

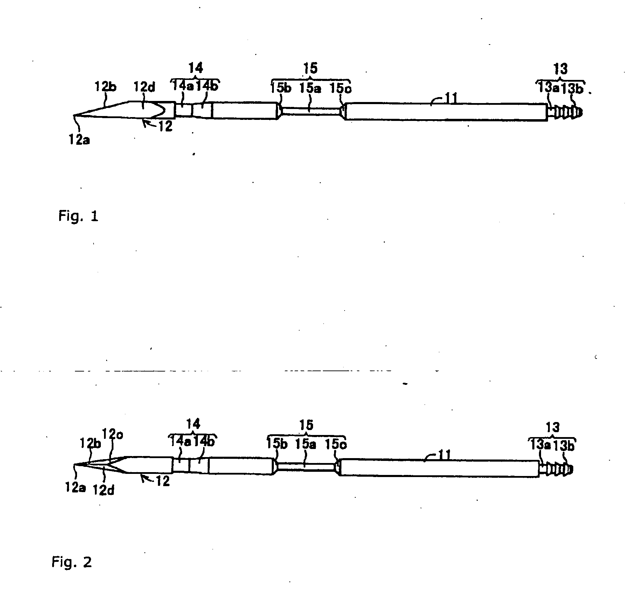

The puncture part 12 is, as shown in Fig. 2, formed at a vertex part 12a, the tip part of which is sharp-pointed, and is comprised of a needle-shaped body, at both sides of which planate side surfaces 12c, 12d are formed so as to form a ridge line part 12b inclining from the vertex part 12a toward the rear upper part. Hereinafter, the present invention will be descried referencing the puncture part 12 side as the front, the connection part 13 side as the rear, the vertex part 12a side of the ridge line part 12b in Fig. 1 as the downside, opposite to the vertex part 12a of the ridge line part 12b as upside.

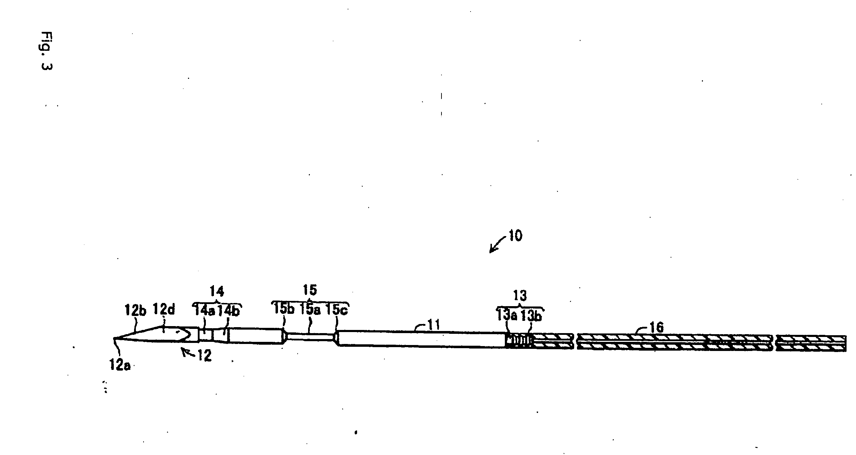

Moreover, the connection part 13 was, as shown in Fig. 3, provided for connecting the medical needle 10 to a drainage tube 16, and comprised of a bar-shaped part 13a having a smaller diameter than that of the needle's main body 11, multiple of retaining protrusions 13b formed on the peripheral surface of the bar-shaped part 13a, keeping the intervals in the longitudinal direction. This retaining protrusions 13b are comprised of the truncated ring-shaped protrusion, the outer peripheral surface of which is tapered in such a manner that the diameter of the front side portion is larger than that of that of the back side portion. Consequently, when the medical needle 10 is connected to the drainage tube 16 by pressing the drainage tube 16 onto the back of the connection part 13 so as to cover the connection part 13, the inner surface of the drainage tube 16 moves forward gliding over the tapered surface of the retaining protrusions 13b to connect the drainage tube 16 to the connection part 13. Moreover, once the drainage tube 16 is connected to the connection part 13, the front end peripheral portion of each of the retaining protrusions 13b acts as resistance, thereby the drainage tube 16 cannot be easily disengaged from the connection part 13.

The engaging stepped part 14 is positioned at the puncture part 12 side, and is comprised of an engaging concave part 14a having a diameter somewhat smaller than that of the rear end part of the puncture part 12 and a tapered part 14b formed at the needle's main body 11 side. The tapered part 14b is formed from the front end part of the needle's main body 11 toward the rear end part of the engaging concave part 14a , with the diameter becoming gradually smaller from the front end part of the needle's main body 11 toward the rear end part side of the engaging concave part 14. The thin part 15 is comprised of a bar-shaped flexible part 15a formed almost the same size as the bar-shaped part 13a of the connection part 13 and a tapering parts 15b , 15c formed at both end sides of the flexible part 15a. The diameter of the tapered part 15b grows larger frontward of the front end part of the flexible part 15a, and the diameter of the tapered part 15c grows larger rearward of the rear end part of the flexible part 15a.

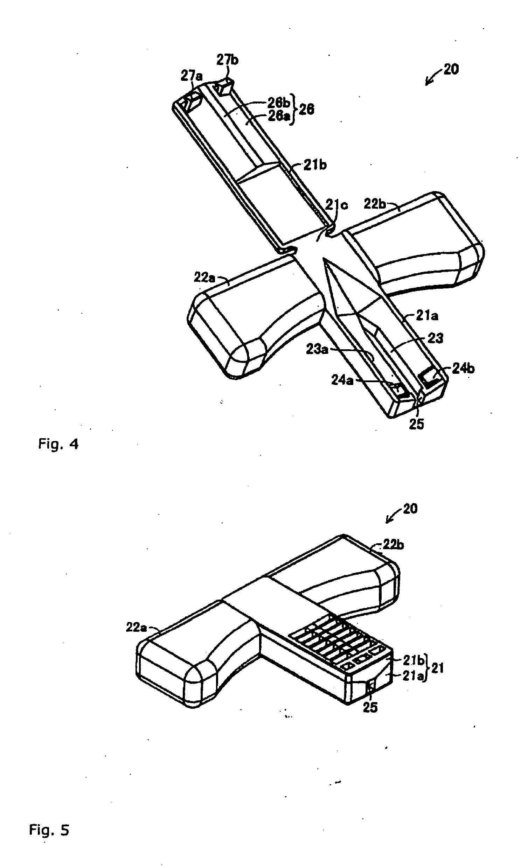

The puller 20 is formed by integral molding a resin material, and as shown in Fig. 4 and Fig. 5, is formed so as to be a truncated cross-shaped when developed, and to be a truncated T-shaped when folded. This puller 20 is comprised of the pair of grippers 22a, 22b formed at a needlepoint coating part 21 and both sides of the needlepoint coating part 21. A needlepoint coating part 21 is structured by hinge-connecting the pair of the coating members consisting of a coating part main body 21a and a lid part 21b at a connecting edge part 21c provided at one end (the top-left side of Fig. 5), allowing to move forward/backward respectively by rotating around the connecting edge part 21c.

The coating part main body 21a is formed in a elongated thick truncated plate-shape extending from one end to the other end, and a needlepoint accommodating concave part 23 capable of accommodating the puncture part 12 of the medical needle 10 is formed with the upper surface (upper surface in the state in Fig. 4) opened from the other end toward the one end side. This needlepoint accommodating concave part 23 is formed so as to be able to accommodate the puncture part 12 with the upper side thereof facing downward (with the top and bottom of Fig. 1 reversed). And, when the puncture part 12 was accommodated in the needlepoint accommodating concave part 23, the puncture part 12 is brought into contact with the wall surface of the needlepoint accommodating concave part 23 in a truncate position without jolt.

Moreover, a needlepoint-shape tapered surface 23a is formed around the needlepoint accommodating concave part 23, spread out so as to enlarge the shape of the needlepoint accommodating concave part 23, on the upper surface of the coating part main body 21a. This needlepoint-shape tapered surface 23a is comprised of the inclined surface, the height of which is gradually reduced from the upper surface of the coating part main body 21a (rim side portion) down toward the needlepoint accommodating concave part 23. Moreover, engaging concave parts 24a, 24b are formed at both side portions of the needlepoint accommodating concave part 23 in the other end side of the needlepoint-shape tapered surface 23a. And, an engaging narrow part 25, the width of which is smaller than the width of the needlepoint accommodating concave part 23 is formed at the portion corresponding to the needlepoint accommodating concave part 23 in the other end of the coating part main body 21a. This engaging narrow part 25 is formed by passing through between the needlepoint accommodating concave part 23 of the coating part main body 21a and outside, and the width is set so as to be smaller than that of the rear end part of the puncture part 12, but to be slightly larger than the engaging concave part 14a of the engaging stepped part 14.

The lid part 21b is comprised of the elongated plate body capable of covering the upper surface of the coating part main body 21a by rotating around the connecting edge part 21c, and both sides of the connecting edge part 21c side portion are formed in planar shape. And, the other end side portion (bottom-right side portion in Fig. 5) is formed to be a concavoconvex surface by forming a multiple of groove parts at regular intervals, and at the inner surface, a convex surface 26 in which the center side of axial direction is protruded is formed at a portion opposite to the needlepoint-shape tapered surface 23a. This convex surface 26 is formed on a tapered surface 26a, both side portions in the width direction of which are capable of interfacing to the needlepoint-shape tapered surface 23a respectively, and the centre part between both the tapered surface 26 side portions is formed to be an elongated the plane 26b.

Therefore, when the upper surface of the coating part main body 21a is covered with the lid part 21b, the side portion of the connecting edge part 21c in the inner surface of the lid part 21b is interfaced with the upper surface of the connecting edge part 21c side of the coating part main body 21a, and at the same time, the tapered surface 26a is interfaced to the needlepoint-shape tapered surface 23a, thereby the upper surface opening of the plane 26b blocks (closes) the needlepoint accommodating concave part 23. Moreover, the other end part of the plane 26b blocks (closes) the opened upper part of the engaging narrow part 25. The needlepoint accommodation part of this invention is comprised of the needlepoint accommodating concave part 23 and the plane 26b, and the engaging part or the concave part of this invention is comprised of the engaging narrow part 25 and the other end part of the plane 26b. And, engaging protrusion parts 27a, 27b capable of engaging to the engaging concave parts 24a, 24b respectively are formed at the other end side portion of both the tapered surface 26a side portions. The chuck part of this invention is comprised of these engaging concave parts 24a, 24b and the engaging protrusion parts 27a, 27b.

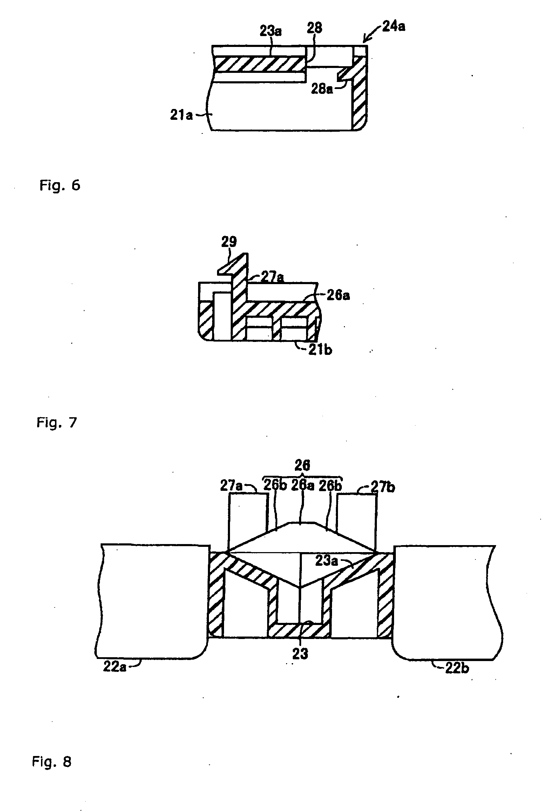

The section of the part on which the engaging concave part(s) 24a (, 24b) in the coating part main body 21a is formed, can appear as shown in Fig. 6, and the section of the part on which the engaging protrusion part(s) 27a (, 27b) in the lid part 21b is formed, can appear as shown in Fig. 7. More specifically, the engaging concave part(s) 24a (, 24b) is comprised of the concave part provided with a small projection 28a at the other end edge part of an opening 28 provided at the needlepoint-shape tapered surface 23a, and the engaging protrusion part(s) 27a (, 27b) is comprised of an elastic protrusion comprising a small projection 29 capable of engaging to the small projection 28a of the engaging concave part(s) 24a (, 24b).

Moreover, the tip-surface of the small projection 29 is formed tapered, and when the upper surface of the coating part main body 21a is covered with the lid part 21b, the engaging protrusion part(s) 27a (, 27b) is while being bowed, penetrating into the inside of the opening 28, and at where the small projection 29 runs past the small projection 28a, the engaging protrusion part(s) 27a (, 27b) returns to the original state, thereby the small projection 29 and the small projection 28a can be engaged. Hereby, the coating part main body 21a and the lid part 21b can be maintained in the folded condition. Moreover, a view of the nearly central section in the longitudinal direction of the coating part main body 21a observed at the angle from the other end side toward one end side is shown in Fig. 8.

The grippers 22a, 22b are extending so as to be perpendicularly intersected with the coating part main body 21a to the different opposite directions respectively from both sides of the connecting edge part 21c side portion of the coating part main body 21a, and the one edge part thereof is formed linearly and the other edge part thereof is formed in a curved shape with center side (the coating part main body 21a side) depressed, so as to be easily grasped by hand. Moreover, the step lowering the upper surface of the coating part main body 21a is provided between the upper surface of the grippers 22a, 22b and the upper surface of the coating part main body 21a. With this step, the height difference between the upper surface of the lid part 21b and the upper surface of the grippers 22a, 22b can be eliminated when the upper surface of the coating part main body 21a is covered by the lid part 21b, thereby the surface of the puller 20 can be In the planar state.

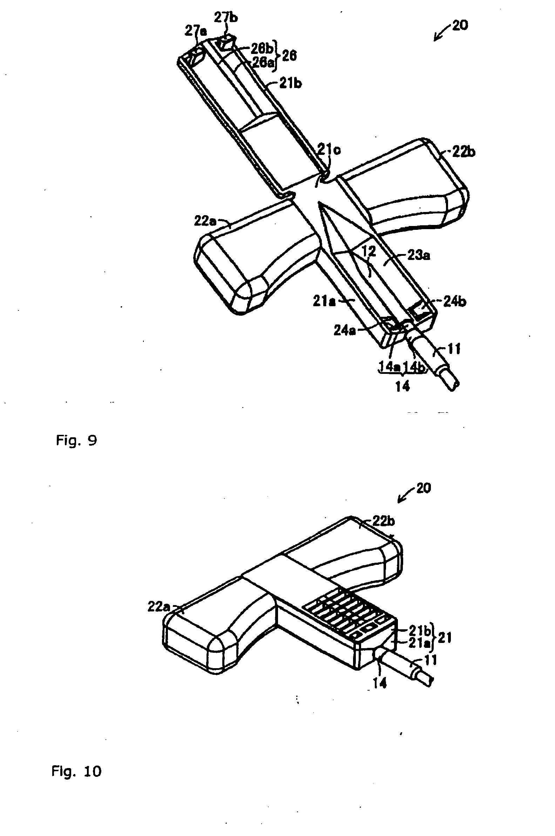

In this structure, when attaching the drainage tube 16 to the wound site of a patient (not shown), firstly, as shown in Fig. 3, the tip part of the drainage tube 16 is connected to the connection part 13 of the medical needle 10. Then, the medical needle 10, with the puncture part 12 first, is inserted through the incision site toward the wound site in the inside of a patient body, and thereafter the puncture part 12 is exteriorized through a predetermined part of the body. In this case, as required, the thin part 15 is bent in such a manner that the medical needle 10 can puncture the appropriate area of the wound site. And, at where the puncture part 12 of the medical needle 10 was exteriorized, the puller 20 is attached to the puncture part 12. In this case, as shown in Fig. 4, the puncture part 12 is inserted in the needlepoint accommodating concave part 23 of the needlepoint coating part 21 in a developed state, with the engaging concave part 14a of the engaging stepped part 14 being positioned to the engaging narrow part 25 so as to be in the sate in Fig. 9.

And, the lid part 21b is rotated to cover the upper surface of the coating part main body 21a, and the lid part 21b is pressed against the coating part main body 21a, so that the engaging protrusion part 27a is engaged to the engaging concave part 24a, and at the same time, the engaging protrusion part 27b is engaged to the engaging concave part 24b. Hereby, the puncture part 12 of the medical needle 10 is coated while being solidly engaged to the needlepoint coating part 21 of the puller 20, thereby the state shown in Fig. 10 can be achieved. Then, the grippers 22a, 22b of the puller 20 is grasped and pulled by hand to exteriorize the medical needle 10.

And, when the tip side portion of the drainage tube 16 is exteriorized, and the rear end part of the drainage tube 16 is positioned to the wound site, the pulling operation should be stopped, and then the drainage tube 16 is cut by a pair of scissors at the connection part side portion with the medical needle 10. Then, the cut portion of the drainage tube 16 is connected to a given aspirator (not shown). And, by actuating the aspirator, the wound fluid can be aspirated through the drainage tube 16 using the aspirator. Moreover, the used medical needle 10 can be discarded with the puller 20 still attached.

As described above, the puller 20 according to the present embodiment is comprised of: the needlepoint coating part 21 for coating the puncture part 12 of the medical needle 10; the engaging part comprised of the engaging narrow part 25 for engaging to the engaging concave part 14a of the engaging stepped part 14 formed at the back side of the puncture part 12 and the other end part of the plane 26b , and the grippers 22a, 22b. Consequently, when pulling out the medical needle 10 from a body, the puller 20 can be attached to the puncture part 12, and the grippers 22a, 22b can be grasped and manipulated, thereby the medical needle 10 can be easily pulled out from a patient body without a risk of user's hand from slipping or to accidentally touch the puncture part 12, which may cause injuries during the operation.

Moreover, in this case, the engaging part comprised of the engaging narrow part 25 of the puller 20 and the other end part of the plane 26b is engaged to the engaging stepped part 14 of the medical needle 10, thereby the puller 20 can no longer be accidentally disengaged from the medical needle 10 when pulling out the puller 20, and thereby the medical needle 10 can be securely pulled out from a body. In addition, the used medical needle 10 can be discarded with the puller 20 still attached to the puncture part 12, thereby neither the removal of the puller 20 from the medical needle 10 nor the attachment of a special cap for disposal and the like to the puncture part 12 is eliminated. Consequently, extra pats for disposal such as a cap, as well as the attaching operations thereof are eliminated.

Moreover, the needlepoint coating part 21 may be comprised of the coating part main body 21a and the lid part 21b hinge-connected at the connecting edge part 21c, the coating part main body 21a and the lid part 21b may be folded together so as to interleave the puncture part 12, thereby the puller 20 can be attached to the medical needle 10, allowing easy attachment of the puller 20 to the medical needle 10. In addition, because of the structure that the needlepoint accommodating concave part 23 is formed on the concave part corresponding to the shape of the puncture part 12, the upper surface of this needlepoint accommodating concave part 23 is blocked (closed) with the plane 26b of the lid part 21b, the puncture part 12 seizes to jolt in the needlepoint accommodating concave part 23, and the puller 20 can now be more solidly attached to the medical needle 10.

Moreover, the puller 20 may now be easily engaged to the medical needle 10, since the coating part main body 21a and the lid part 21b can be folded together by simply positioning the engaging narrow part 25 at the engaging stepped part 14 of the medical needle 10 allowing easier manipulation. In addition, a risk of the puller 20 disengaging from the puncture part 12 can be eliminated and the puller 20 can remain attached to the medical needle by simply pressing the coating part main body 21a and the lid part 21b one another and by engaging the engaging concave part 24a and the engaging protrusion part 27a with the engaging concave part 24b and the engaging protrusion part 27b respectively,

### (The Second Embodiment)

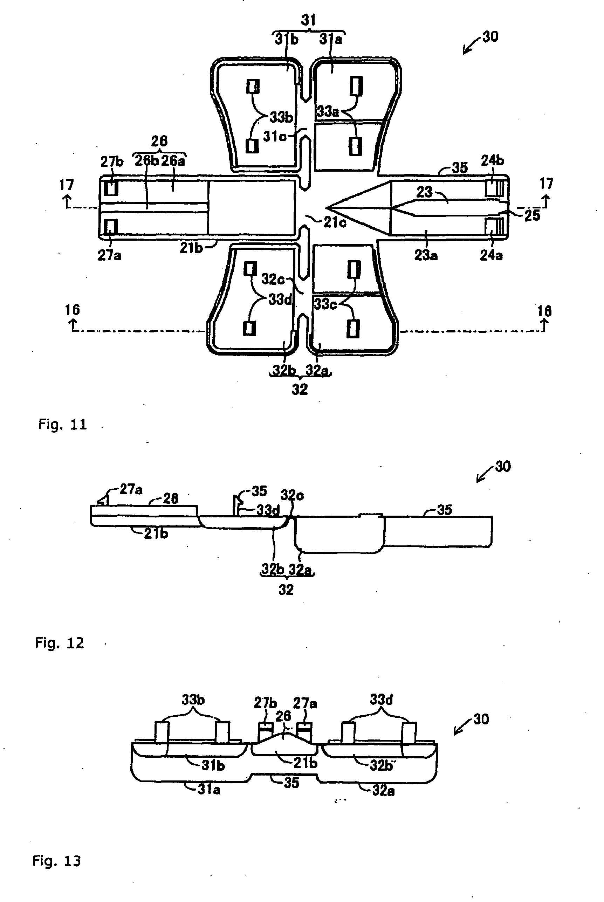

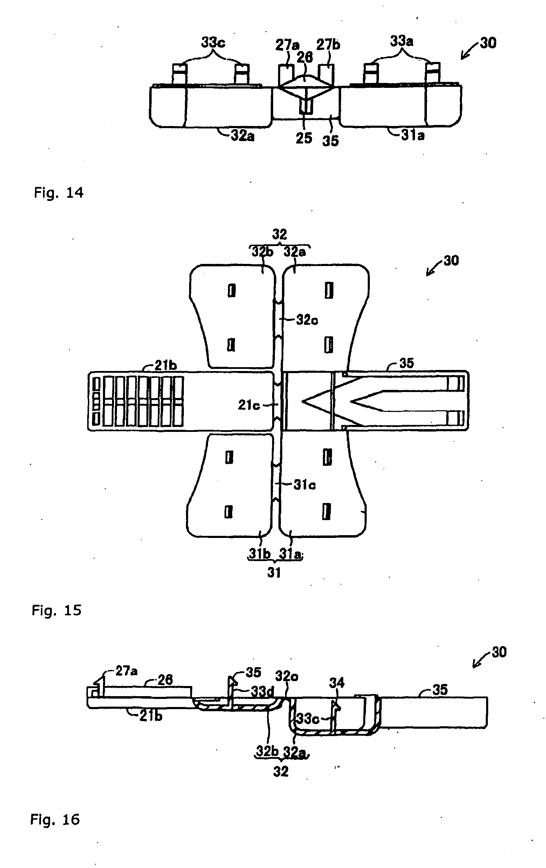

In Fig. 11 or Fig. 15, a developed view of a puller 30 according to the second embodiment of this invention is shown. This puller 30, in which grippers 31, 32 are not individual protrusions, but are 2 pieces of lid-shaped members and are stacked and assembled together so as to be formed to have an outer shape to be the same as the aforementioned grippers 22a, 22b. More specifically, the gripper 31 is structured by hinge-connecting the pair of lid-shaped members consisting of the gripper main body 31a and the lid part 31b at the connection edge part 31c, allowing to move forward/backward respectively by rotating around the connecting edge part 31c.

The gripper 32 is formed so as to be vertically symmetric with the gripper 31 (vertically symmetric in the state of in Fig. 11), and as with the gripper 31, is structured by hinge-connecting the pair of lid-shaped members consisting of a gripper main body 32a and a lid part 32b at a connection edge part 32c, allowing to move forward/backward respectively by rotating around the connecting edge part 32c. And, engaging protrusion parts 33a-33d are formed respectively on the inner surfaces of the gripper main body 31a and the lid part 31b, and of the gripper main body 32a and the lid part 32b (2 parts each). 2 pieces of the engaging protrusion part 33a of the gripper main body 31a and 2 pieces of the engaging protrusion part 33c of a gripper main body 32a are respectively formed facing in the same direction, and 2 pieces of the engaging protrusion part 33b of the lid part 31b and 2 pieces of the engaging protrusion part 33d of the lid part 32b are respectively formed facing in the same direction.

Among these grippers, the section of the gripper 32 including the outer engaging protrusion part 33c and the engaging protrusion part 33d are shown in Fig. 16. More specifically, the engaging protrusion part 33c is comprised of the elastic protrusion on which a small projection 34 is formed at the tip part, and the tip-surface of the small projection 34 is formed tapered. Moreover, the engaging protrusion part 33d is also comprised of the elastic protrusion on which a small projection 35 is formed at the tip part, and the tip-surface of the small projection 35 is formed tapered. And, when the lid part 32b is rotated around the connection edge part 32c to be stacked on the upper surface of the gripper main body 32a, the engaging protrusion part 33c and the engaging protrusion part 33d are bent and stacked together while pressing the respective tapered surfaces.

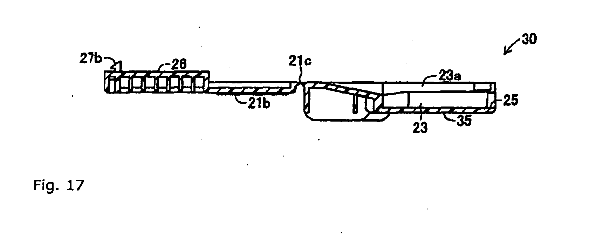

And, at where the small projection 35 runs past the small projection 34, the engaging protrusion part 33c and the engaging protrusion part 33d return to the original state, thereby the small projection 34 and the small projection 35 are engaged. Hereby, the assembled state of the gripper main body 32a and the lid part 32b can be maintained. Moreover, the gripper main body 31a and the lid part 31b are also assembled in the same manner. Meanwhile, in this puller 30, the gripper main bodies 31a, 32a are connected to the coating part main body 35 and the lid parts 31b, 32b are connected only to the gripper main bodies 31a, 32a. Moreover, a 17-17 section in Fig. 11 is shown in Fig. 17.

Other structures/configuratians of this puller 30 are the same as the puller 20 of the aforementioned first embodiment. Thus, the identical notations are assigned to the identical parts and the detailed descriptions are omitted. According to this embodiment, the grippers 31, 32 can be formed into a empty box-shape, thereby the molding material usage and the associated costs can be reduced, and at the same time, the weight of the puller 30 can also be reduced. Other functions and effects of the puller 30 according to this embodiment are the same as the puller 20 of the aforementioned first embodiment.

Moreover, the present invention is not limited to the aforementioned embodiments, may be optionally modified and implemented accordingly within the scope of the invention. For example, in the aforementioned embodiment, while the engaging stepped part 14 of the medical needle 10 was comprised of the thin part, it may also be comprised of the protrusion part. Moreover, in each of the aforementioned embodiments, while the pullers are provided with respective grippers, these pullers may also be comprised only of the needlepoint coating part comprising the engaging part consisting of an engaging narrow part and the like. In this case, the needlepoint coating part may be grasped and pulled out by hand. In addition, in the aforementioned second embodiment, while the lid part 31b and the lid part 32b are not connected to the lid part 21b, these parts may also be connected together into one integrated piece.

### [Brief Description of the Drawings]

[Fig. 1] A side view showing the medical needle to which the puller according to the first embodiment of this invention is attached.
[Fig. 2] A front view showing the medical needle.
[Fig. 3] A side view showing the medical needle connected to the drainage tube.
[Fig. 4] A perspective view showing the puller developed.
[Fig. 5] A perspective view showing the puller assembled.
[Fig. 6] A cross-section view showing the engaging concave part.
[Fig. 7] A cross-section view showing the engaging protrusion.
[Fig. 8] A cross-section view showing the coating part main body.
[Fig. 9] A perspective view showing the puncture part of the medical needle set on the puller of Fig. 4.
[Fig. 10] A perspective view showing the medical needle attached to the puller.
[Fig. 11] A plane view showing the puller according to the second embodiment developed.
[Fig. 12] A front view showing the puller according to the second embodiment developed.
[Fig. 13] A left side view showing the puller according to the second embodiment developed.
[Fig. 14] A right side view showing the puller according to the second embodiment developed.
[Fig. 15] A bottom view showing the puller according to the second embodiment developed.
[Fig. 16] The 16-16 cross-section of Fig. 11.
[Fig. 17] The 17-17 cross-section of Fig. 11.

### [Description of Notation]

10 Medical needle, 12 Puncture part, 13 Connection part, 14 Engaging stepped part, 14a Engaging concave part, 16 Drainage tube, 20, 30 Puller, 21 Needlepoint coating part, 21a, 35 Coating part main body, 21b Lid part, 21c Connection edge parts, 22a, 22b, 31, 32, Gripper, 23 Needlepoint accommodation concave parts, 24a, 24b Engaging concave part, 25 Engaging narrow part, 26b Planes, 27a, 27b Engaging protrusion part.

## Claims

1. A medical needle puller to be attached to a medical needle, in which a connection part for connecting a drainage tube placed at a postsurgical wound site is formed at a rear end part, a puncture part is formed at a tip part, and at the same time an engaging stepped part is formed at the back side of the aforementioned puncture part, comprising:
an engaging part engaging to the aforementioned engaging stepped part; and
a needlepoint coating part for coating the aforementioned puncture part comprising a pair of coating members hinge-connected at one edge and folded together, between which a needlepoint accommodation part for accommodating the aforementioned puncture part is formed, whereby said needlepoint accommodation part is ***characterised in***
a needlepoint accommodating concave part (23) corresponding to the shape of the puncture part and a plane (26b) adapted to close said needlepoint accommodating concave part and bring said medical needle into contact with a wall surface of said needlepoint accommodating concave part.

2. The medical needle puller according to claim 1, wherein a gripper for pulling the aforementioned puller by hand is provided when the aforementioned medical needle is pulled out from the aforementioned wound site together with the aforementioned puller, while attaching the aforementioned puller to the puncture part of the aforementioned medical needle.

3. The medical needle puller according to claim 2, wherein the aforementioned needlepoint coating part is formed so as to be extending along the extending direction of the aforementioned puncture part, and wherein the aforementioned gripper is comprised of a pair of protrusions extending to different directions respectively so as to be perpendicularly intersecting with the aforementioned needlepoint coating part from both sides of the width direction of the aforementioned needlepoint coating part.

4. The medical needle puller according to either one of claim 1 or 3, wherein the aforementioned engaging part is comprised of a concave part formed at a predetermined part of the jointed surface joined when the aforementioned pair of coating members are folded together, passing through the aforementioned needlepoint coating part from inside to outside.

5. The medical needle puller according to claim 4, wherein the pair of the coating members structuring the aforementioned needlepoint coating part are provided with a chuck part for maintaining the aforementioned pair of coating members in the folded condition.

## Patentansprüche

1. Medizinischer Nadelzieher zur Befestigung an einer medizinischen Nadel, in der ein Befestigungsabschnitt zum Befestigen einer Dränageleitung, die an einer postoperativen Wundstelle angeordnet ist, an einem rückwärtigen Endabschnitt gebildet ist, wobei ein Punktierabschnitt an einem Spitzenabschnitt gebildet ist und zugleich ein abgesetzter Eingriffsabschnitt an der Rückseite des vorgenannten Punktierabschnitts gebildet ist, aufweisend:
einen Eingriffabschnitt, der in den vorgenannten abgesetzten Eingriffabschnitt eingreift; und
einen Nadelspitzenumhüllungsabschnitt um den vorgenannten Punktierabschnitt zu umhüllen, der ein Paar von Umhüllungselementen, die an einer Kante gelenkig verbunden und zusammengeklappt sind, zwischen denen ein Nadelspitzenaufnahmeabschnitt zum Aufnehmen des vorgenannten Punktierabschnitts gebildet ist, aufweist, wobei der Nadelspitzenaufnahmeabschnitt ***gekennzeichnet ist durch***
einen konkaven Nadelspitzenaufnahmeabschnitt (23), der der Form des Punktierabschnitts entspricht, und eine Ebene (26b), die geeignet ist, den konkaven Nadelspitzenaufnahmeabschnitt zu verschließen und die medizinische Nadel in Kontakt mit einer Wandoberfläche des konkaven Nadelspitzenaufnahmeabschnitts zu bringen.

2. Medizinischer Nadelzieher nach Anspruch 1, wobei ein Greifer zum von Hand Ziehen des vorgenannten Nadelziehers bereitgestellt ist, wenn die vorgenannte medizinische Nadel aus der vorgenannten Wundstelle zusammen mit dem vorgenannten Nadelzieher herausgezogen wird, während der vorgenannte Nadelzieher an dem Punktierabschnitt der vorgenannten medizinischen Nadel anliegt.

3. Medizinischer Nadelabzieher nach Anspruch 2, wobei der vorgenannte Nadelspitzenumhüllungsabschnitt so geformt ist, dass er sich entlang der Erstreckungsrichtung des vorgenannten Punktierabschnitts erstreckt und wobei der vorgenannte Greifer aus einem Paar von Vorsprüngen gebildet ist, die sich jeweils in verschiedene Richtungen erstrecken, so dass sie rechtwinklig den vorgenannten Nadelspitzenumhüllungsabschnitt von beiden Seiten in Richtung der Breite des vorgenannten Nadelspitzenumhüllungsabschnitts kreuzen.

4. Medizinischer Nadelzieher nach einem der beiden Ansprüche 1 oder 3, wobei der vorgenannte Eingriffabschnitt aus einem konkaven Abschnitt gebildet ist, der an einem vorbestimmten Abschnitt der gelenkig verbundenen Oberfläche ausgebildet ist, die zusammengefügt wird, wenn das vorgenannte Paar Umhüllungselemente zusammengeklappt wird, wobei sie von innen durch den vorgenannten Nadelspitzenumhüllungsabschnitt nach außen treten.

5. Medizinischer Nadelzieher nach Anspruch 4, wobei das Umhüllungselementepaar, das den vorgenannten Nadelspitzenumhüllungsabschnitt bildet, mit einem Spannabschnitt ausgestattet ist, um das vorgenannte Umhüllungselementepaar in der zusammengeklappten Lage zu halten.

## Revendications

1. Crochet d'aiguille médicale à fixer à une aiguille médicale, où une partie de connection pour relier un tube de drainage placé à un site de plaie post-chirurgicale est formée à une partie d'extrémité arrière, une partie de ponction est formée à la partie de pointe et, en même temps, une partie étagée d'engagement est formée au côté arrière de la partie de ponction précitée, comprenant:
une partie d'engagement venant en prise avec la partie étagée d'engagement précitée; et
une partie de recouvrement de la pointe d'aiguille pour revêtir la partie de ponction précitée comprenant deux éléments de revêtement reliés d'une manière articulée à un bord et pliés ensemble, entre lesquels une partie de logement de pointe d'aiguille pour recevoir la partie de ponction précitée est formée, par quoi ladite partie de logement de pointe d'aiguille est **caractérisée par**
une partie concave de réception de pointe d'aiguille (23) correspondant à la forme de la partie de ponction et un plan (26b) apte à fermer ladite partie concave de réception de pointe d'aiguille et à amener ladite aiguille médicale en contact avec une surface de paroi de la partie concave de réception de pointe d'aiguille.

2. Crochet d'aiguille médicale selon la revendication 1, où une prise pour tirer le crochet précité à la main est réalisée lorsque l'aiguille médicale précitée est retirée du site de plaie précité conjointement avec le crochet précité tout en fixant le crochet précité à la partie de ponction de l'aiguille médicale précitée.

3. Crochet d'aiguille médicale selon la revendication 2, où la partie de recouvrement de pointe d'aiguille précitée est formée de manière à s'étendre le long de la direction d'extension de la partie de ponction précitée, et où la prise précitée est constituée d'une paire de saillies s'étendant dans des directions différentes respectivement de manière à se croiser perpendiculairement avec la partie de recouvrement de pointe d'aiguille précitée depuis les deux côtés de la direction de largeur de la partie de recouvrement de pointe d'aiguille précitée.

4. Crochet d'aiguille médicale selon l'une quelconque des revendications 1 ou 3, où la partie d'engagement précitée est constituée d'une partie concave formée à une partie prédéterminée de la surface jointe qui est jointe lorsque la paire précitée d'éléments de recouvrement sont pliés ensemble, en passant à travers la partie de revêtement de pointe d'aiguille précitée de l'intérieur à l'extérieur.

5. Crochet d'aiguille médicale selon la revendication 4, où la paire d'éléments de recouvrement structurant la partie de revêtement de pointe d'aiguille précitée sont munis d'une partie de serrage pour maintenir la paire d'éléments de recouvrement précitée dans l'état plié.
